# EUROPEAN PATENT APPLICATION

(11) **EP 4 542 563 A1**
(43) Date of publication of application: **23.04.2025**
(21) Application number: 23826912.0
(22) Date of filing: 29.05.2023
(51) Int. Cl.: G16H 20/60

(54) **INFORMATION PROCESSING DEVICE, INFORMATION PROCESSING METHOD, AND INFORMATION PROCESSING PROGRAM**

(30) Priority: 20.06.2022 JP 2022098673
(71) Applicant: ZOZO, Inc., Chiba-shi, Chiba, 263-0023 (JP)
(72) Inventor: MIYAZAWA, Takahiro, Chiba-shi, Chiba 263-0023 (JP); MAKINO, Yohei, Chiba-shi, Chiba 263-0023 (JP); YAMADA, Takayasu, Chiba-shi, Chiba 263-0023 (JP); FUJII, Yuki, Chiba-shi, Chiba 263-0023 (JP); ONO, Kengo, Chiba-shi, Chiba 263-0023 (JP); HIGASHIHARA, Eri, Chiba-shi, Chiba 263-0023 (JP); ANDO, Fuminori, Chiba-shi, Chiba 263-0023 (JP); YOSHIOKA, Shunya, Chiba-shi, Chiba 263-0023 (JP); HORI, Miyuki, Chiba-shi, Chiba 263-0023 (JP)
(74) Representative: Hoffmann Eitle
(86) International application number: PCT/JP2023/019916
(87) International publication number: WO 2023/248724

(57) **Abstract**

An information processing apparatus according to the present application includes an acquisition unit that acquires state information on a state of a user (for example, body information on a body of the user, a service that is available to the user, attribute information, or the like), a setting unit that sets an index that is achievable in the state of the user with respect to a change in a body shape of the user, based on the state information that is acquired by the acquisition unit, and a providing unit that provides information on the index that is set by the setting unit to the user.

## Description

### Field

The present invention relates to an information processing apparatus, an information processing method, and an information processing program.

### Background

Conventionally, a technology for supporting a user to transform into a target body shape has been proposed. As one example of the technology as described above, a configuration has been disclosed that sets target body shape data that indicates a target body shape of a user, estimates future body shape data that indicates a body shape of the user after a lapse of a predetermined period based on measured body data of the user, calculates a cost needed to achieve the target body shape data based on the estimated future body shape data, and displays the cost on a display.

### Citation List

### Patent Literature

Japanese Laid-open Patent Publication No. 2021-142000

### Summary

### Technical Problem

However, in the technology as described above, it is not possible to present an appropriate index in accordance with a state of a user.

For example, in the technology as described above, a configuration that adopts, as the target body shape data, a certain body shape that is selected from among a plurality of different body shapes that correspond to an age, a height, and gender of the user, and it is not possible to present an appropriate index in accordance with the state of the user.

The present application has been conceived in view of the above circumstances, and an object of the present application is to present an appropriate index in accordance with a state of a user.

### Solution to Problem

Information processing apparatus according to the present disclosure, includes an acquisition unit that acquires state information on a state of a user, a setting unit that sets an index that is achievable in the state of the user with respect to a change in a body shape of the user, based on the state information that is acquired by the acquisition unit, and a providing unit that provides information on the index that is set by the setting unit to the user.

### Advantageous Effects of Invention

According to one aspect of one embodiment, it is possible to present an appropriate index in accordance with a state of a user.

### Brief Description of Drawings

FIG. 1 is a diagram illustrating a configuration example of an information processing system 1 according to one embodiment.
FIG. 2 is a diagram illustrating an example of information processing according to one embodiment.
FIG. 3 is a diagram illustrating a configuration example of an information processing apparatus 10 according to one embodiment.
FIG. 4 is a diagram illustrating an example of a region information database 31 according to one embodiment.
FIG. 5 is a diagram illustrating an example of a user information database 32 according to one embodiment.
FIG. 6 is a flowchart illustrating an example of the flow of information processing according to one embodiment.
FIG. 7 is a hardware configuration diagram illustrating an example of a computer that implements functions of the information processing apparatus 10. Description of Embodiments

Modes (hereinafter, referred to as "embodiments") for carrying out an information processing apparatus, an information processing method, and an information processing program according to the present application will be described in detail below with reference to the drawings. Meanwhile, the information processing apparatus, the information processing method, and the information processing program according to the present application are not limited by the embodiments below. In addition, in each of the embodiments described below, the same components are denoted by the same reference symbols, and repeated explanation will be omitted.

### Embodiment

### 1. Configuration of information processing system

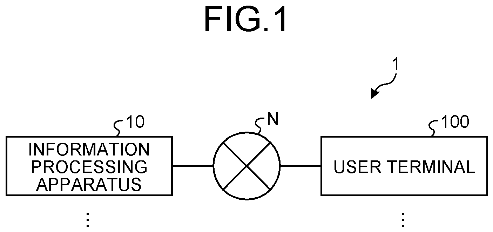

An information processing system 1 according to one embodiment will be described below. FIG. 1 is a diagram illustrating a configuration example of the information processing system 1 according to one embodiment. As illustrated in FIG. 1, the information processing system 1 includes an information processing apparatus 10 and a user terminal 100. The information processing apparatus 10 and the user terminal 100 are communicably connected to each other in a wired or wireless manner via a predetermined communication network (network N). Meanwhile, the information processing system 1 illustrated in FIG. 1 may include a plurality of the information processing apparatuses 10 and a plurality of the user terminals 100.

The information processing apparatus 10 is an information processing apparatus that presents an appropriate index in accordance with a state of a user with respect to a change in a body shape of the user (in other words, diet (training)), and implemented by, for example, a server device, a cloud system, or the like. For example, the information processing apparatus 10 captures an image (performs imaging) of a peripheral body that is dressed in clothes for measuring a body size (for measuring a body shape size) by the user terminal 100, receives, as body information (measurement information) on a body of the user, a user body model (for example, a 3D model) that is generated based on dots (markers) that are attached to the clothes for measuring the body size, and provides a service (hereinafter, a diet service (training service)) that presents information on whether or not the body of the user is approaching a set goal.

Meanwhile, the information processing apparatus 10 may be an apparatus that distributes, to the user terminal, information to be displayed on applications that are related to various kinds of services and that are installed in the user terminal 100. Furthermore, the information processing apparatus 10 may be a server that distributes data of an application.

Moreover, the information processing apparatus 10 may function as a distribution apparatus that distributes control information to the user terminal 100. Here, the control information is described in, for example, script language, such as JavaScript (registered trademark), or style sheet language, such as Cascading Style Sheets (CSS). Meanwhile, it may be possible to regard an application that is distributed from the information processing apparatus 10 as the control information.

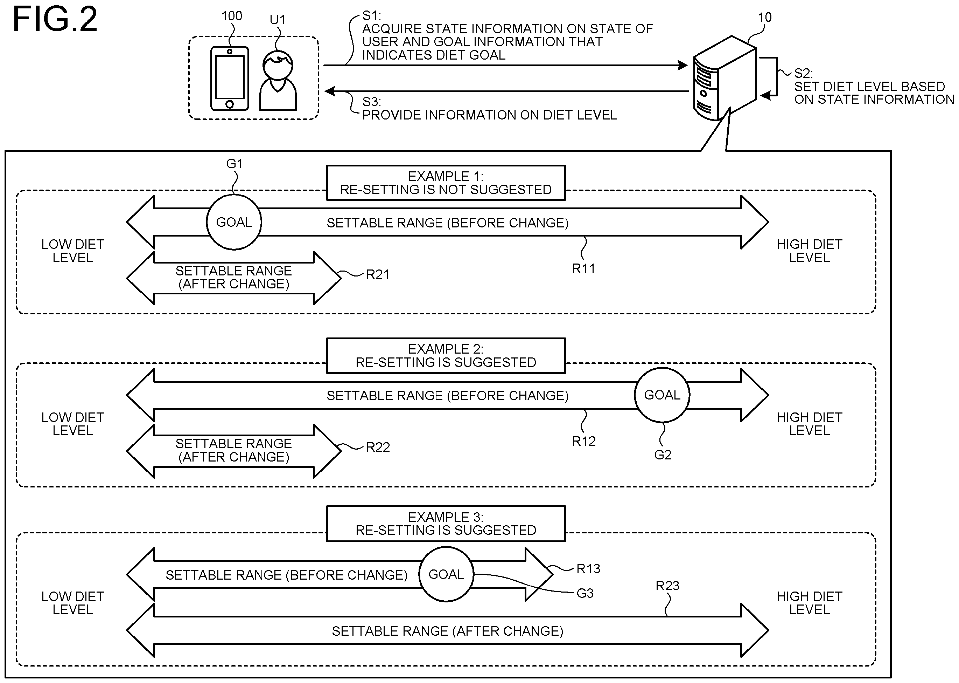

The user terminal 100 is an information processing apparatus that is used by a user. The user terminal 100 is implemented by, for example, a smartphone, a tablet terminal, a notebook Personal Computer (PC), a desktop PC, a mobile phone, a Personal Digital Assistant (PDA), or the like. Further, the user terminal 100 displays information that is distributed from the information processing apparatus 10, a server device that provides a predetermined service, or the like on a web browser or an application. Meanwhile, in the example illustrated in FIG. 2, a case is illustrated in which the user terminal 100 is a smartphone.

### 2. Example of information processing

An example of information processing that is implemented by the information processing apparatus or the like according to the present embodiment will be described below with reference to FIG. 2. FIG. 2 is a diagram illustrating an example of information processing according to one embodiment. Meanwhile, in the following descriptions, it is assumed that the user terminal 100 is used by a user (user U1) that is identified by a user ID of "UID#1". Further, in the following descriptions, in some cases, the user terminal 100 may be regarded as the user U1. In other words, in the following, the user U1 may be replaced with the user terminal 100.

Furthermore, in the following descriptions, it is assumed that the information processing apparatus 10 collects (acquires) information, such as map information on each of regions or information on a service that is available in each of the regions and that supports diet (for example, a fitness club (a fitness gym, a training gym, or a sports gym), a dietary guidance service, or the like; hereinafter, may be described as a "support service"), and manages the information in a storage unit of the subject apparatus. For example, the information processing apparatus 10 stores therein regional information that includes identification information (region ID) for identifying each of the regions, location information (a location, a range, or the like) of each of the regions, map information in each of the regions, service information (for example, a location, a content of a support service to be provided, or the like) on a support service that is available in each of the regions. Meanwhile, a range with an arbitrary area may be appropriately set as a region. For example, the region may be a grid square (mesh) that has the same size and that is divided based on latitude and longitude. In other words, the region may be a region that is segmented by a regional mesh. In this case, a regional mesh code may be used as the regional ID. Meanwhile, the above is one example, and therefore, the region is not limited to the regional mesh, and may be set based on various kinds of information. For example, the region may be set based on an administrative division, such as a "town", a "district", a "city", or a "prefecture". Furthermore, the region may be set based on a facility, such as a commercial facility (a shopping center, a home center, an outlet mall, an underground shopping area, or the like), or may be set based on a street or a shopping street.

First, the information processing apparatus 10 acquires state information on a state of the user U1 and goal information that indicates a diet goal (Step S1). For example, the information processing apparatus 10 acquires, as the state information, a place of residence or a work place of the user U1 (in other words, a base of the user U1), a support service that is used (subscribed) by the user U1, a usage detail of the support service (for example, a frequency of use of the fitness club or the dietary guidance service, a usage time, a content of a training menu that is provided by the fitness club, a dietary guidance that is provided by the dietary guidance service), or the like from the user terminal 100, the server device that is managed by the provider who provides the support service, or the like. Further, the information processing apparatus 10 acquires, as the state information, a body model of the user U1, body information that indicates a height or a weight of the user U1, or the like from the user terminal 100.

Furthermore, the information processing apparatus 10 acquires, as the goal information, information on a weight, a waist size, a weight or a body shape of a celebrity or an athlete (an exemplary weight or an exemplary body shape), or the like that is adopted as the diet goal by the user U1 from the user terminal 100.

Subsequently, the information processing apparatus 10 sets a diet level (in other words, a range of a possible weight loss) as an index that is achievable in the current state of the user U1 based on the state information on the user U1 (Step S2). For example, the information processing apparatus 10 sets the diet level of the user U1 based on information, such as the body model of the user U1, the weight of the user U1, the support service that is used by the user U1, the usage detail (a frequency of use or the like) of the support service that is used, or a support service that is not subscribed among support services that are available in the place of residence or the work place of the user U1. As a specific example, the information processing apparatus 10 sets the diet level of the user U1 based on information that is based on the body model, the weight, or the usage detail of the support service of the user U1 (for example, calorie consumption that is based on exercise intensity in the fitness club or calorie intake from a meal that is based on the dietary guidance that is provided by the dietary guidance service), or information that is based on a support service that is not subscribed by the user U1 among support services that are available in the place of residence or the work place of the user U1 (for example, calorie consumption that is based on exercise intensity in the fitness club or calorie intake from a meal that is based on the dietary guidance that is provided by the dietary guidance service). More specifically, the information processing apparatus 10 sets, as the diet level of the user U1, any of a plurality of levels from a diet level "1 (weight loss is extremely small (for example, weight loss by 5% and waist size deduction by 5 centimeters (cm))) to a diet level "10 (weight loss is extremely large (for example, weight loss by 30% and waist size reduction by 20 cm)).

Subsequently, the information processing apparatus 10 provides information on the diet level to the user terminal 100 (Step S3). For example, when information on a goal setting that is recommended in accordance with the diet level (for example, a weight or a waist size that are recommended as a goal or information on a weight, a body shape, or the like of a celebrity or an athlete) or the diet goal that is indicated by the goal information exceeds the diet level that is set by the information processing apparatus 10, the information processing apparatus 10 provides information for suggesting re-setting of a goal (for example, information for proposing to lower the goal), information for improving the diet level (for example, information on increase in exercise intensity in the fitness club, improvement in the training menu that is provided by the fitness club, improvement in the dietary guidance that is provided by the dietary guidance service, information for proposing subscription of an unsubscribed support service (a support service that collaborates with the diet service or the like)), or the like.

Meanwhile, at Step S2, the information processing apparatus 10 may set a diet level (hereinafter, described as a "diet level #2") for a case where the user U1 improves the usage detail of the support service that is used by the user U1 in addition to a diet level (hereinafter, described as a "diet level #1") that is based on the usage detail of the support service that is currently used by the user U1, or may set a diet level that is based on the support service that is currently used by the user U1 and a support service that is not subscribed by the user U11 among support services that are available in the place of residence or the work place of the user U1 (in other words, a diet level for a case where an unsubscribed support service is used in addition to the currently used support service; hereinafter, described as a "diet level #3").

Further, when the diet goal that is indicated by the goal information exceeds the diet level #1, but does not exceed the diet level #2, the information processing apparatus 10 provides information for proposing improvement in the usage detail of the support service (for example, information on increase in the exercise intensity in the fitness club, improvement in the training menu that is provided by the fitness club, improvement in the dietary guidance that is provided by the dietary guidance service, or the like). When the diet goal that is indicated by the goal information exceeds the diet level #1, but does not exceed the diet level #3, the information processing apparatus 10 provides information for proposing subscription of an unsubscribed support service.

Furthermore, the information processing apparatus 10 may set the diet level of the user U1 based on the state information on the state of the user U1, and thereafter allow the user U1 to set a diet goal within a range of the set diet level. For example, when the diet level of the user U1 is set to "5", the information processing apparatus 10 allows the user U1 to set a diet goal in the range from the diet level "1" to the diet level "5" (hereinafter, may be described as a "settable range").

In other words, the information processing apparatus 10 is able to limit a goal that can be set by the user U1 in accordance with the set diet level. Meanwhile, the information processing apparatus 10 may provide information on a support service that may achieve the goal, based on the body information on the user and the diet goal that is set by the user. Specifically, the information processing apparatus 10 provides information on a support service that may achieve the goal, based on a change in a weight or a body shape of a different user who has used each of support services (achievement of a different user), the body information on the user, and the diet goal that is set by the user.

Furthermore, the information processing apparatus 10 may re-set the diet level in accordance with a goal achievement rate that indicates a level of progress that the body of the user has reached the diet goal that is set by the user. Moreover, the information processing apparatus 10 may re-set the diet level in accordance with the diet goal that is set by the user and a goal achievement rate with respect to the goal. For example, when the diet level of the user is set to "5", when the diet goal that is set by the user corresponds to the diet level "5", and when the goal achievement rate is equal to or larger than a first threshold (for example, equal to or larger than 90%), the information processing apparatus 10 re-sets the diet level to a higher level (for example, re-sets the diet level to "6" or "7"). Furthermore, when the diet level of the user is set to "5", when the diet goal that is set by the user corresponds to the diet level "5", and when the goal achievement rate is equal to or smaller than a second threshold (for example, equal to or smaller than 10%), the information processing apparatus 10 re-sets the diet level to a lower level (for example, re-sets the diet level to "4" or "3").

Here, when the state information (for example, the support service that is used by the user U1 or a support service that is not subscribed among support services that are available in the place of residence or the work place of the user U1) is changed due to the reason caused by the user, such as relocation of the user U1, or due to the reason caused by the service-side, such as start or stop of the support service, it may be possible to suggest re-setting of the diet goal in accordance with the changed diet level.

For example, as illustrated in Example 1, when a diet goal G1 of the user U1 is set in a range of a settable range R11 (for example, in the range of the diet level of "1" to "10") based on the state information that is not yet changed and when the goal G1 is in a range of a settable range R21 (for example, in the range of the diet level of "1" to "3") based on the changed state information, the information processing apparatus 10 does not suggest re-setting of the diet goal.

In contrast, as illustrated in Example 2, when a diet goal G2 of the user U1 is set in a range of a settable range R12 (for example, in the range of the diet level of "1" to "10") that is based on the state information that is not yet changed and when the goal G2 exceeds the range of the settable range R22 (for example, in the range of the diet level of "1" to "3") that is based on the changed state information, the information processing apparatus 10 provides information for suggesting re-setting of the diet goal within the range of the settable range R22.

Furthermore, as illustrated in Example 3, when a diet goal G3 of the user U1 is set in a range of a settable range R13 (for example, in the range of the diet level of "1" to "6") that is based on the state information that is not yet changed, when the goal G3 is in a range of a settable range R23 (for example, in the range of the diet level of "1" to "10") that is based on the changed state information, and the range of the settable range R23 exceeds the range of the settable range R13, the information processing apparatus 10 provides information for suggesting re-setting of the diet goal in a range that exceeds the settable range R13 and that is within the range of the settable range R23.

In other words, the information processing apparatus 10 determines whether or not to suggest re-setting of the diet goal in accordance with a relationship between the diet goal that is set by the user (goal information) and the changed settable range.

Meanwhile, the information processing apparatus 10 may determine whether or not to suggest re-setting of the diet goal in accordance with the goal achievement rate that indicates a level of progress that the body of the user has reached the diet goal that is set by the user. For example, the information processing apparatus 10 does not suggest re-setting of the diet goal when the goal achievement rate is equal to or larger than a predetermined threshold (for example, equal to or larger than 95%), and suggests re-setting of the diet goal when the goal achievement rate is smaller than the predetermined threshold. This is because when the body of the user is very close to the diet goal (for example, when the goal achievement rate is equal to or larger than 95%), even if the support service or the like that is used by the user is changed, the diet goal that is set is not excessive.

Furthermore, when suggesting re-setting of the diet goal, the information processing apparatus 10 may provide information that indicates a reason for the settable range, together with the information for suggesting re-setting. For example, when providing information for suggesting raising of the diet goal, the information processing apparatus 10 provides, as the information that indicates the reason for the settable range, information indicating that it is possible to increase the exercise intensity in the fitness club in which the user has joined, improve the training menu that is provided by the fitness club, or improve the dietary guidance that is provided by the dietary guidance service, or information indicating that a support service that is not subscribed by the user is present among support services that are available to the user. Furthermore, when providing the information for suggesting lowering of the diet goal, the information processing apparatus 10 provides, as the information that indicates a reason for the settable range, information indicating that it is impossible to increase the exercise intensity in the fitness club in which the user has joined, improve the training menu that is provided by the fitness club, or improve the dietary guidance that is provided by the dietary guidance service, or information indicating that a support service that is not subscribed by the user is absent among support services that are available to the user.

As described above, the information processing apparatus 10 according to one embodiment is able to present an achievable range in accordance with the support service that is used by the user or the available support service, and prevent setting of an excessive diet goal. In other words, the information processing apparatus 10 according to one embodiment is able to an appropriate index in accordance with the state of the user.

Furthermore, the information processing apparatus 10 according to one embodiment provides information for proposing subscription of a support service that collaborates with a diet service among support services that are available in the place of residence or the work place of the user and that are not subscribed by the user. With this configuration, the information processing apparatus 10 according to one embodiment is able to send the user to the support service that collaborates with the diet service, so that it is possible to closely link the information (for example, acquire information based on the usage detail of the support service), and it is possible to provide more accurate information to the user.

Meanwhile, the information processing apparatus 10 according to one embodiment may transmit information (goal information) that indicates the diet goal of the user who is sent to the support service or information (difference information) that indicates a difference between the body of the user and the diet goal to the server device that is managed by the provider who provides the support service. Furthermore, the information processing apparatus 10 according to one embodiment may calculate a service charge for sending of the user or a service charge for transmission of the goal information or the difference information on the user, and transmit the service charge to the server device that is managed by the provider who provides the support service.

### 3. Configuration of information processing apparatus

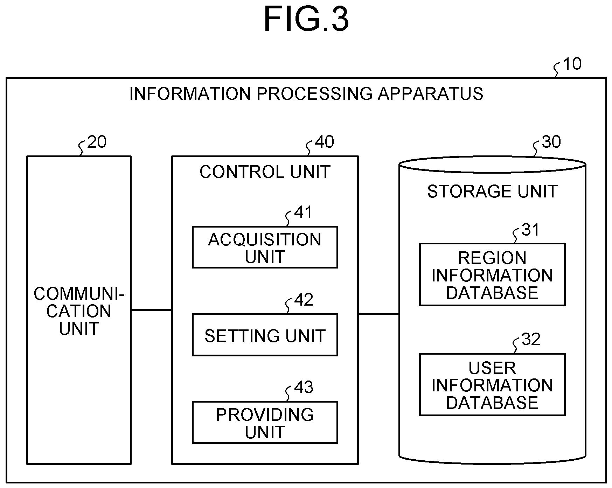

A configuration of the information processing apparatus 10 will be described below with reference to FIG. 3. FIG. 3 is a diagram illustrating a configuration example of the information processing apparatus 10 according to one embodiment. As illustrated in FIG. 3, the information processing apparatus 10 includes a communication unit 20, a storage unit 30, and a control unit 40.

### Communication unit 20

The communication unit 20 is implemented by, for example, a Network Interface Card (NIC) or the like. Further, the communication unit 20 is connected to the network N in a wired or wireless manner, and transmits and receives information to and from the user terminal 100 or the like.

### Storage unit 30

The storage unit 30 is, for example, a semiconductor memory device, such as a Random Access Memory (RAM) or a Flash Memory, or a storage device, such as a hard disk or an optical disk. As illustrated in FIG. 3, the storage unit 30 includes a region information database 31 and a user information database 32.

### Region information database 31

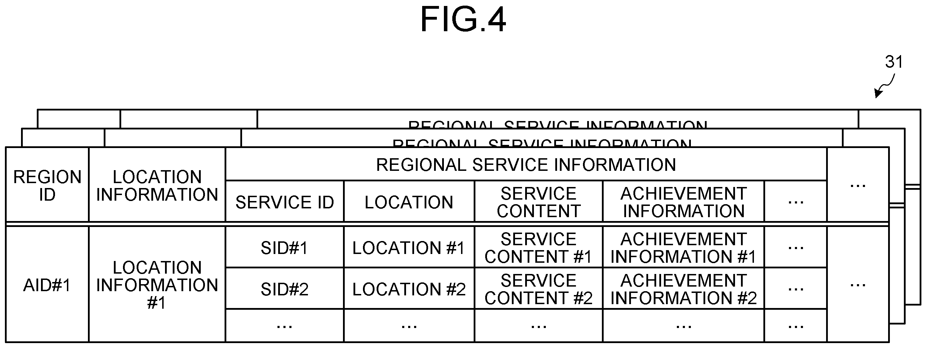

The region information database 31 stores therein various kinds of information on each of regions. An example of the information that is stored in the region information database 31 will be described below with reference to FIG. 4. FIG. 4 is a diagram illustrating an example of the region information database 31 according to one embodiment. In the example illustrated in FIG. 4, the region information database 31 includes items such as a "region ID", "location information", and "regional service information".

The "region ID" indicates identification information for identifying a region. The "location information" indicates information on a location, a range, or the like of the region. The "regional service information" indicates information on a support service that is available in the region, and includes items such as a "service ID", a "location", a "service content", and "achievement information". The "service ID" indicates identification information for identifying a support service. The "location" indicates a location of a facility or the like that provides the support service. The "service content" indicates a content of the support service to be provided. The "achievement information" indicates information on a change in a weight or a body shape of a user who has used the support service.

Specifically, FIG. 4 illustrates an example in which the location information on a region that is identified by a region ID of "AID#1" is "location information #1", a support service that is available in the region is identified by a service ID of "SID#1", a location of a facility that provides the support service is a "location #1", a content of the support service is a "service content #1", and the achievement information is "achievement information #1".

### User information database 32

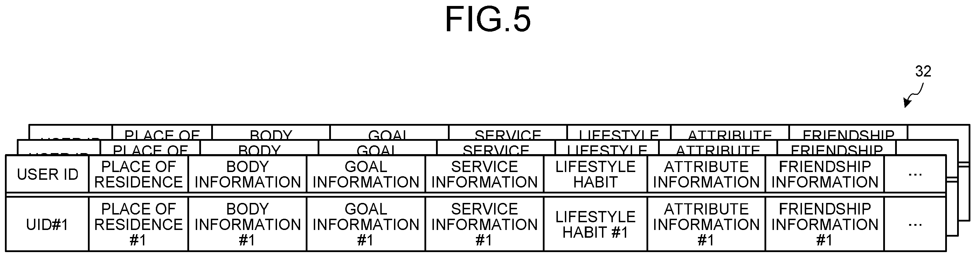

The user information database 32 stores therein various kinds of information on a user. An example of the information that is stored in the user information database 32 will be described below with reference to FIG. 5. FIG. 5 is a diagram illustrating an example of the user information database 32 according to one embodiment. In the example illustrated in FIG. 5, the user information database 32 includes items such as a "user ID", a "place of residence", "body information", "goal information", "service information", a "lifestyle habit", "attribute information", and "friendship information". Meanwhile, it may be possible to include an item such as a "work place" in which information (a region ID or the like) that indicates a work place of the user is stored. Further, it may be possible to include an item such as a "place of school" in which information (a region ID or the like) that indicates a place of a school of the user is stored.

The "user ID" indicates identification information for identifying a user. In the "place of residence", information (a region ID or the like) that indicates a place of residence of the user is stored. The "body information" indicates information on a body of the user, and stores therein a body model of the user, information indicating the height, the weight, blood pressure, a blood sugar level, or the like of the user. The "goal information" indicates a diet goal that is set by the user. The "service information" indicates information on a support service that is used by the user, and, for example, the service ID, a usage state of the support service (for example, a frequency of use, a date and time of use, a usage time, a content of a training menu that is provided by a fitness club, or a content of a dietary guidance that is provided by a dietary guidance service), or the like is stored. The "lifestyle habit" indicates a lifestyle habit of the user (for example, an early bird, a night owl, or an exercise habit, such as exercising or not exercising). The "attribute information" indicates an attribute of the user (for example, a demographic attribute or a psychographic attribute). The "friendship information" indicates information (a user ID or the like) on a different user who has a predetermined relationship in a predetermined service (for example, a user who is followed or a user who has a mutual link in an SNS service).

Specifically, FIG. 5 illustrates an example in which a place of residence of the user who is identified by the user ID of "UID#1" is a "place of residence #1", the body information is "body information #1", the goal information is "goal information #1", the service information is "service information #1", the lifestyle habit is a "lifestyle habit #1", the attribute information is "attribute information #1", and the friendship information is "friendship information #1".

### Control unit 40

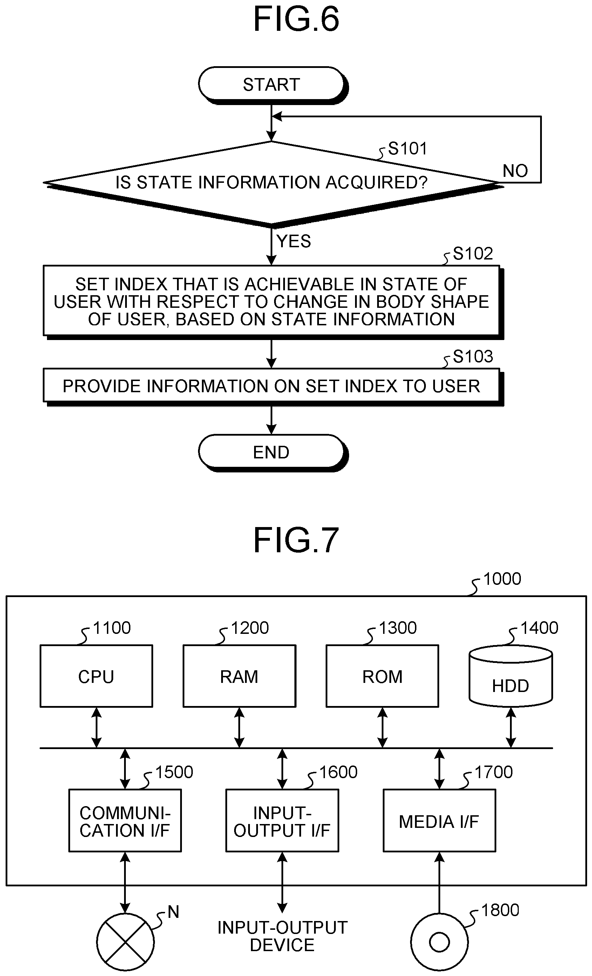

The control unit 40 is a controller (controller) and implemented by, for example, causing a Central Processing Unit (CPU), a Micro Processing Unit (MPU), or the like to execute various kinds of programs that are stored in an internal storage device of the information processing apparatus 10 by using a RAM as a work area. Furthermore, the control unit 40 is a controller and implemented by, for example, an integrated circuit, such as an Application Specific Integrated Circuit (ASIC) or a Field Programmable Gate Array (FPGA). The control unit 40 according to one embodiment includes, as illustrated in FIG. 6, an acquisition unit 41, a setting unit 42, and a providing unit 43, and implements functions and operation of information processing to be described below.

### Acquisition unit 41

The acquisition unit 41 acquires state information on a state of the user. For example, in the example illustrated in FIG. 2, the acquisition unit 41 acquires state information on a state of the user U1 from the user terminal 100, a server device that is managed by a provider who provides the support service, or the like, and stores the state information in the user information database 32.

Furthermore, the acquisition unit 41 may acquire state information that indicates body information on a body of the user. For example, in the example illustrated in FIG. 2, the acquisition unit 41 acquires the body model of the user U1 or the body information that indicates the height, the weight, or the like of the user U1 from the user terminal 100.

Moreover, the acquisition unit 41 may acquire state information that indicates service information on a service that is available to the user. For example, in the example illustrated in FIG. 2, the acquisition unit 41 acquires the support service that is used by the user U1, a usage detail of the support service, or the like from the user terminal 100, the server device that is managed by the provider who provides the support service, or the like.

Furthermore, the acquisition unit 41 may acquire state information that indicates service information on a change in a body shape that is achieved by using the service. For example, the acquisition unit 41 acquires service information (achievement information) that indicates information on a change in a weight of the user, a change in various sizes of the body, or the like by using the support service (that is, achievement by the support service).

Moreover, the acquisition unit 41 may acquire state information that indicates service information on a frequency of use of the service by the user. For example, in the example illustrated in FIG. 2, the acquisition unit 41 acquires service information that indicates a frequency of use of the support service.

Furthermore, the acquisition unit 41 may acquire state information that indicates service information on an available service in accordance with the lifestyle habit of the user. For example, the acquisition unit 41 acquires service information on an available support service in accordance with the information (for example, whether the lifestyle habit indicates a night owl, an early bird, exercising, or not exercising) that is collected by the wearable device that is worn on the user. As a specific example, when the lifestyle habit of the user indicates a "night owl", the acquisition unit 41 acquires service information on a support service that provides a service until late hours (for example, a fitness club that opens 24 hours). Furthermore, when the lifestyle habit of the user indicates an "early bird", the acquisition unit 41 acquires service information on a support service that provides a service from early hours (for example, a fitness club that opens at 6 o'clock in the morning or a fitness club that opens 24 hours). Moreover, when the lifestyle habit of the user indicates "not exercising", the acquisition unit 41 acquires service information on a support service that provides a service other than exercise (for example, a dietary guidance service or an electronic commerce service that sells dietary supplement). Meanwhile, the acquisition unit 41 may acquire state information that indicates the lifestyle habit of the user based on input information that is input by the user.

Furthermore, the acquisition unit 41 may acquire state information that indicates service information on a service that is available in the place of residence or the work place of the user. For example, in the example illustrated in FIG. 2, the acquisition unit 41 acquires service information on each of support services that are available in a region in which the user U1 lives or a region in which the user U1 works. Meanwhile, the acquisition unit 41 may acquire state information that indicates service information on a support service that is available in a place of a school of the user.

Moreover, the acquisition unit 41 may acquire state information that indicates attribute information on the user. For example, the acquisition unit 41 acquires attribute information that indicates a demographic attribute or a psychographic attribute of the user.

Furthermore, the acquisition unit 41 may acquire state information that indicates information on a different user who has a predetermined relationship with the user. For example, the acquisition unit 41 acquires information on a different user who is followed by the user in an SNS service or a different user who has a mutual link or a mutual follow. As a specific example, the acquisition unit 41 acquires information that is posted by a different user in an SNS service (for example, information on diet, information on use of a support service, or the like) or information that indicates a frequency of posting the information.

Moreover, the acquisition unit 41 may further acquire goal information that is set by the user and that indicates a goal related to a change in the body shape. For example, the acquisition unit 41 acquires, from the user terminal 100, information, such as a weight or a waist size that is adopted as the diet goal by the user U1, a weight or a body shape of a celebrity or an athlete, or the like.

### Setting unit 42

The setting unit 42 sets an index that is achievable in the state of the user with respect to a change in the body shape of the user, based on the state information that is acquired by the acquisition unit 41. For example, in the example illustrated in FIG. 2, the setting unit 42 refers to the region information database 31 and the user information database 32, and sets the diet level that is achievable in the current state of the user U1 based on the state information on the user U1.

Furthermore, the setting unit 42 may set an index based on the body information. For example, in the example illustrated in FIG. 2, the setting unit 42 sets the diet level that is achievable in the current state of the user U1 based on the body model of the user U1 or the body information that indicates the height or the weight of the user U1.

Moreover, the setting unit 42 may set an index based on the service information. For example, in the example illustrated in FIG. 2, the setting unit 42 sets the diet level of the user U1 based on information that is based on the usage detail of the support service or information related to a support service that is not subscribed by the user U1 among support services that are available in the place of residence or the work place of the user U1.

Meanwhile, the setting unit 42 may set an index based on a change in the body shape in a case where the support service is used (in other words, achievement of the support service). For example, the setting unit 42 sets the diet level of the user who is using the support service or the diet level of the user in a case where the user subscribes the support service, based on a change in a weight or a body shape of a different user who has used the support service. Furthermore, for example, the setting unit 42 may set the diet level of the user, based on a change in a weight or a body shape of a different user who has used the support service, among users who are using the support service, a diet level of the user who has a certain weight or a certain body shape that is similar to a weight or a body shape of the different user before the different user uses the support service.

Moreover, the setting unit 42 may set an index based on the service information on an available support service in accordance with the lifestyle habit of the user. For example, the setting unit 42 sets a diet level of the user in a case where the user subscribes the support service, based on information on a support service that is available in accordance with the lifestyle habit of the user among support services that are available in the place of residence or the work place of the user and that are not subscribed by the user.

Furthermore, the setting unit 42 may set an index based on the attribute information. For example, the setting unit 42 sets the diet level based on whether or not the user has a child. As a specific example, when the user has an infant (in other words, in a case of a user who may not have a time to use a support service, such as a fitness club), the setting unit 42 sets a lower diet level than a user who does not have an infant. Moreover, for example, the setting unit 42 sets the diet level based on the nationality of the user (difference in culture or a lifestyle habit), gender, or an age.

Furthermore, the setting unit 42 may set an index based on information on a different user. For example, the setting unit 42 sets a higher diet level of the user with an increase in a posting frequency of information on diet by a different user who is followed by the user or a different user who has a mutual link or a mutual follow in an SNS service (in other words, when the user is more encouraged to maintain motivation for diet due to posts by a different user).

Moreover, when the state information is changed, the setting unit 42 may re-set the index related to a change in the body shape of the user based on the changed state information. For example, in the example illustrated in FIG. 2, when the state information is changed due to the reason caused by the user, such as relocation of the user U1, or due to the reason caused by the service-side, such as start or stop of the support service, the setting unit 42 re-sets the diet level based on the changed state information.

### Providing unit 43

The providing unit 43 provides information on the index that is set by the setting unit 42 to the user. For example, in the example illustrated in FIG. 2, the providing unit 43 provides information on the diet level to the user terminal 100.

Furthermore, the providing unit 43 may provide information on setting of a goal related to a change in the body shape of the user, in accordance with the index. For example, the providing unit 43 provides, as a goal that is recommended to the user in accordance with the diet level, a weight, a waist size, information on a weight, a body shape, or the like of a celebrity or an athlete, or the like.

Moreover, the providing unit 43 may provide information on a service in accordance with the index. For example, in the example illustrated in FIG. 2, when the diet goal that is indicated by the goal information exceeds the set diet level, the providing unit 43 provides information for suggesting re-setting of a goal (for example, information for proposing to lower the goal), information non increase in the exercise intensity in the fitness club, improvement in the training menu that is provided by the fitness club, or improvement in the dietary guidance that is provided by the dietary guidance service, information for proposing subscription of an unsubscribed support service (a support service that collaborates with the diet service or the like), or the like.

Furthermore, when a difference between the index that is set by the setting unit 42 and the goal that is indicated by the goal information meets a predetermined condition, the providing unit 43 may provide information for proposing to change the goal that is indicated by the goal information. For example, in the example illustrated in FIG. 2, when the diet goal that is indicated by the goal information exceeds the set diet level, the providing unit 43 provides information for proposing to lower the goal.

Meanwhile, in the example illustrated in FIG. 2, when the diet goal that is indicated by the goal information is lower than the set diet level, the providing unit 43 may provide information for proposing to raise the goal. In this case, the providing unit 43 may provide information on a support service that corresponds to the changed goal (for example, information on increase in the exercise intensity in the fitness club, improvement in the training menu that is provided by the fitness club, or improvement in the dietary guidance that is provided by the dietary guidance service, or information for proposing subscription of an unsubscribed support service (a support service that collaborates with the diet service or the like)).

Furthermore, when a difference between the index that is re-set by the setting unit 42 and the goal that is indicated by the goal information meets a predetermined condition, the providing unit 43 may provide information for proposing to change the goal that is indicated by the goal information. For example, as illustrated in the second example in FIG. 2, when the diet goal G2 of the user U1 is set in the range of the settable range R12 based on the state information that is not yet changed and the goal G2 exceeds the range of the settable range R22 that is based on the changed state information, the providing unit 43 provides information for suggesting raising of the diet goal within the settable range R22. Moreover, as illustrated in the third example in FIG. 2, when the diet goal G3 of the user U1 is set in the range of the settable range R13 that is based on the state information that is not yet changed, when the goal G3 is in the range of the settable range R23 that is based on the changed state information, and the range of the settable range R23 exceeds the range of the settable range R13, the setting unit 42 provides information for suggesting re-setting of the diet goal in a range that exceeds the settable range R13 and that is within the range of the settable range R23.

### 4. Flow of information processing

A flow of information processing performed by the information processing apparatus 10 according to one embodiment will be described with reference to FIG. 6. FIG. 6 is a flowchart illustrating an example of the flow of information processing according to one embodiment.

As illustrated in FIG. 6, the information processing apparatus 10 determines whether or not the state information is acquired (Step S101). When the state information is not acquired (Step S101; No), the information processing apparatus 10 waits until the state information is acquired.

In contrast, when the state information is acquired (Step S101; Yes), the information processing apparatus 10 sets an index that is achievable in the state of the user with respect to a change in the body shape of the user, based on the state information (Step S102). Subsequently, the information processing apparatus 10 provides information on the set index to the user (Step S103), and the process is terminated.

### 5. Modification

The embodiment as described above illustrates one example, and various changes and modifications may be made.

### 5-1. Body information

In one embodiment as described above, the example has been described in which the acquisition unit 41 acquires the body model of the user or the body information that indicates the height or the weight of the user, but the functions of the acquisition unit 41 are not limited to the example as described above, and it may be possible to acquire arbitrary information on the body of the user. For example, the acquisition unit 41 may acquire information, such as blood pressure or a blood sugar level of the user, as the body information. In this case, the providing unit 43 may provide information on a service that corresponds to the blood pressure or the blood sugar level. For example, when the blood pressure of the user is equal to or larger than a predetermined threshold and it is dangerous to exercise excessively, the providing unit 43 provides information for proposing to use a fitness club (for example, information for proposing exercise at low intensity) or information for proposing to use a dietary guidance service. Furthermore, when the blood sugar level of the user is equal to or larger than a predetermined threshold, information for proposing to use a fitness club, information for proposing to use a dietary guidance service, or the like is provided.

### 5-2. Goal

In one embodiment as described above, the example has been described in which the acquisition unit 41 acquires a weight or a waist size that is adopted as the diet goal by the user, but the functions of the acquisition unit 41 are not limited to the example as described above. For example, the acquisition unit 41 may acquire various kinds of sizes of the body (for example, a chest circumference (chest), a hip circumference (hip), a thigh circumference, a calf circumference, an arm circumference, or a leg circumference) that are adopted as the diet goal by the user. In this case, the providing unit 43 may provide equipment (including a training menu) that is effective to a part that is adopted as a diet target by the user or information related to a fitness club that includes the equipment.

### 5-3. Mode of processing

Of the processes described in the embodiments, all or part of a process described as being performed automatically may also be performed manually. Alternatively, all or part of a process described as being performed manually may also be performed automatically by known methods. In addition, the processing procedures, specific names, and information including various kinds of data and parameters illustrated in the above-described document and drawings may be arbitrarily changed unless otherwise specified. For example, various kinds of information illustrated in each of the drawings are not limited to the information illustrated in the drawing.

Furthermore, the components of the apparatuses illustrated in the drawings are functionally conceptual and do not necessarily have to be physically configured in the manner illustrated in the drawings. In other words, specific forms of distribution and integration of the apparatuses are not limited to those illustrated in the drawings, and all or part of the apparatuses may be functionally or physically distributed or integrated in arbitrary units depending on various loads or use conditions.

Furthermore, the embodiments as described above may be combined appropriately as long as processing contents do not conflict with each other.

### 6. Effects

As described above, the information processing apparatus 10 according to one embodiment includes the acquisition unit 41, the setting unit 42, and the providing unit 43. The acquisition unit 41 acquires state information on a state of a user. The setting unit 42 sets an index that is achievable in the state of the user with respect to a change in a body shape of the user, based on the state information that is acquired by the acquisition unit 41. The acquisition unit 41 further acquires goal information that indicates a goal that is set by the user in relation to a change of a body shape. The providing unit 43 provides information on the index that is set by the setting unit 42 to the user. Further, the providing unit 43 provides information on setting of the goal in relation to the change in the body shape of the user in accordance with the index. Furthermore, the providing unit 43 provides information on a service in accordance with the index. Moreover, the providing unit 43, when a difference between the index that is set by the setting unit 42 and the goal that is indicated by the goal information meets a predetermined condition, provides information for proposing to change the goal that is indicated by the goal information.

With this configuration, the information processing apparatus 10 according to one embodiment is able to present an achievable range in accordance with the state of the user and prevent setting of an excessive diet goal, so that it is possible to present an appropriate index in accordance with the state of the user.

Furthermore, in the information processing apparatus 10 according to one embodiment, for example, the acquisition unit 41 acquires the state information that indicates body information related to a body of the user. Further, the setting unit 42 sets the index based on the body information. Furthermore, the acquisition unit 41 acquires the state information that indicates service information on a service that is available to the user. Moreover, the setting unit 42 sets the index based on the service information. Furthermore, the acquisition unit 41 acquires the state information that indicates the service information related to a change in a body shape with use of the service. Moreover, the acquisition unit 41 acquires the state information that indicates the service information on a frequency of use of the service by the user. Furthermore, the acquisition unit 41 acquires the state information that indicates the service information on the available service in accordance with a lifestyle habit of the user. Moreover, the acquisition unit 41 acquires the state information that indicates the service information on the service that is available in one of a place of residence and a work place of the user. Furthermore, the acquisition unit 41 acquires the state information that indicates attribute information on the user. Moreover, the setting unit 42 sets the index based on the attribute information. Furthermore, the acquisition unit 41 acquires the state information that indicates information on a different user who has a predetermined relationship with the user. Moreover, the setting unit 42 sets the index based on the information on the different user.

With this configuration, the information processing apparatus 10 according to one embodiment is able to set the diet goal based on various kinds of information on the user, so that it is possible to present an appropriate index in accordance with the state of the user.

Furthermore, in the information processing apparatus 10 according to one embodiment, for example, the setting unit 42, when the state information is changed, re-sets the index related to the change in the body shape of the user based on the changed state information. Moreover, the providing unit 43, when a difference between the index that is re-set by the setting unit 42 and the goal that is indicated by the goal information meets a predetermined condition, provides information for proposing to change the goal that is indicated by the goal information.

With this configuration, the information processing apparatus 10 according to one embodiment is able to re-set an appropriate diet goal in accordance with a change in the state of the user, so that it is possible to improve usability.

### 7. Hardware configuration

Each of the information processing apparatuses 10 according to one embodiment as described above is implemented by, for example, a computer 1000 that is configured as illustrated in FIG. 7. In the following, explanation will be given by adopting the information processing apparatus 10 as an example. FIG. 7 is a hardware configuration diagram illustrating an example of a computer that implements the functions of the information processing apparatus 10. The computer 1000 includes a CPU 1100, a RAM 1200, a Read Only Memory (ROM) 1300, a Hard Disk Drive (HDD) 1400, a communication interface (I/F) 1500, an input-output interface (I/F) 1600, and a media interface (I/F) 1700.

The CPU 1100 operates based on a program that is stored in the ROM 1300 or the HDD 1400, and controls each of the units. The ROM 1300 stores therein a boot program that is executed by the CPU 1100 at the time of activation of the computer 1000, a program that is dependent on hardware of the computer 1000, or the like.

The HDD 1400 stores therein a program that is executed by the CPU 1100, data that is used by the program, or the like. The communication interface 1500 receives data from a different device via a communication network 500 (corresponding to the network N of one embodiment), sends the data to the CPU 1100, and transmits data that is generated by the CPU 1100 to a different device via the communication network 500.

The CPU 1100 controls an output device, such as a display or a printer, and an input device, such as a keyboard or a mouse, via the input-output interface 1600. The CPU 1100 acquires data from the input device via the input-output interface 1600. Further, the CPU 1100 outputs generated data to the output device via the input-output interface 1600.

The media interface 1700 reads a program or data that is stored in a recording medium 1800, and provides the program or the data to the CPU 1100 via the RAM 1200. The CPU 1100 loads the program from the recording medium 1800 onto the RAM 1200 via the media interface 1700, and executes the loaded program. The recording medium 1800 is, for example, an optical recording medium, such as a Digital Versatile Disc (DVD) or a Phase change rewritable Disk (PD), a magneto-optical recording medium, such as a Magneto-Optical disk (MO), a tape medium, a magnetic recording medium, a semiconductor memory, or the like.

For example, when the computer 1000 functions as the information processing apparatus 10, the CPU 1100 of the computer 1000 executes the program that is loaded on the RAM 1200, and implements the functions of the control unit 40. Further, the HDD 1400 stores therein each piece of data that is stored in an internal storage device of the information processing apparatus 10. The CPU 1100 of the computer 1000 reads the program from the recording medium 1800 and executes the program; however, as another example, it may be possible to acquire the program from a different apparatus via a predetermined communication network.

### 8. Others

While some embodiments of the present application have been described in detail above based on the drawings, the embodiments are mere examples, and the present invention may be embodied with various modifications or changes based on knowledge of a person skilled in the art, in addition to the embodiments described in the section of Disclosure of Embodiments.

Furthermore, the configuration of the information processing apparatus 10 as described above may be flexibly changed such that some functions may be implemented by calling an external platform or the like by an Application Programming Interface (API), network computing, or the like.

Moreover, a "unit" recited in the claims may be replaced with a "means", a "circuit", or the like. For example, the receiving unit may be replaced with a receiving means or a receiving circuit.

### Reference Signs List

- 10: information processing apparatus

- 20: communication unit
- 30: storage unit
- 31: region information database
- 32: user information database
- 40: control unit
- 41: acquisition unit
- 42: setting unit
- 43: providing unit
- 100: user terminal

## Claims

1. An information processing apparatus comprising:
an acquisition unit that acquires state information on a state of a user;
a setting unit that sets an index that is achievable in the state of the user with respect to a change in a body shape of the user, based on the state information that is acquired by the acquisition unit; and
a providing unit that provides information on the index that is set by the setting unit to the user.

2. The information processing apparatus according to claim 1, wherein the providing unit provides information on setting of the goal in relation to the change in the body shape of the user in accordance with the index.

3. The information processing apparatus according to claim 1, wherein the providing unit provides information on a service in accordance with the index.

4. The information processing apparatus according to claim 1, wherein
the acquisition unit acquires the state information that indicates body information related to a body of the user, and
the setting unit sets the index based on the body information.

5. The information processing apparatus according to claim 1, wherein
the acquisition unit acquires the state information that indicates service information on a service that is available to the user, and
the setting unit sets the index based on the service information.

6. The information processing apparatus according to claim 5, wherein the acquisition unit acquires the state information that indicates the service information related to a change in a body shape with use of the service.

7. The information processing apparatus according to claim 5, wherein the acquisition unit acquires the state information that indicates the service information on a frequency of use of the service by the user.

8. The information processing apparatus according to claim 5, wherein the acquisition unit acquires the state information that indicates the service information on the available service in accordance with a lifestyle habit of the user.

9. The information processing apparatus according to claim 5, wherein the acquisition unit acquires the state information that indicates the service information on the service that is available in one of a place of residence and a work place of the user.

10. The information processing apparatus according to claim 1, wherein
the acquisition unit acquires the state information that indicates attribute information on the user, and
the setting unit sets the index based on the attribute information.

11. The information processing apparatus according to claim 1, wherein
the acquisition unit acquires the state information that indicates information on a different user who has a predetermined relationship with the user, and
the setting unit sets the index based on the information on the different user.

12. The information processing apparatus according to claim 1, wherein
the acquisition unit further acquires goal information that indicates a goal that is set by the user in relation to a change of a body shape, and
the providing unit, when a difference between the index that is set by the setting unit and the goal that is indicated by the goal information meets a predetermined condition, provides information for proposing to change the goal that is indicated by the goal information.

13. The information processing apparatus according to claim 12, wherein
the setting unit, when the state information is changed, re-sets the index related to the change in the body shape of the user based on the changed state information, and
the providing unit, when a difference between the index that is re-set by the setting unit and the goal that is indicated by the goal information meets a predetermined condition, provides information for proposing to change the goal that is indicated by the goal information.

14. An information processing method that is implemented by a computer, the information processing method comprising:
an acquisition step of acquiring state information on a state of a user;
a setting step of setting an index that is achievable in the state of the user with respect to a change in a body shape of the user, based on the state information that is acquired at the acquisition step; and
a providing step of providing information on the index that is set at the setting step to the user.

15. An information processing program that causes a computer to perform a process comprising:
an acquisition step of acquiring state information on a state of a user;
a setting step of setting an index that is achievable in the state of the user with respect to a change in a body shape of the user, based on the state information that is acquired at the acquisition step; and
a providing step of providing information on the index that is set at the setting step to the user.
